# EUROPEAN PATENT APPLICATION

(11) **EP 2 703 026 A1**
(43) Date of publication of application: **05.03.2014**
(21) Application number: 12182908.9
(22) Date of filing: 04.09.2012
(51) Int. Cl.: A61M 5/32

(54) **Safety needle assembly**

(71) Applicant: Sanofi-Aventis Deutschland GmbH, 65929 Frankfurt am Main (DE)
(72) Inventor: The designation of the inventor has not yet been filed

(57) **Abstract**

Described is a safety needle assembly (1) comprising a drive unit (2) holding a needle (3) having a distal pointed end (3.1), an outer body (4) rotatably coupled to the drive unit (2), a first tube (6) telescopically coupled to the outer body (4) and the drive unit (2), and a second tube (7) telescopically coupled to the first tube (6). In an extended position, the second tube (7) covers the distal pointed end (3.1) of the needle (3), and rotation of the outer body (4) relative to the drive unit (2) causes the first tube (6) and the second tube (7) to retract and expose the distal pointed end (3.1).

## Description

### Technical Field

The invention relates to a safety needle assembly for attaching to an injection device.

### Background of the Invention

Drug delivery devices that contain a selected dosage of a medicament or drug are well known devices for administering the medicament to a patient. Thereby, needle safety has become an important factor to consider when using needles. It is desirable to protect users and health care professionals from unintended needle sticks either with a clean or a contaminated needle.

Safety devices for covering a medical needle of the drug delivery device before and after use are also well known. Typically, a needle shield of the safety device is either manually moved or translated by the action of a relaxing spring and/ or using latches to surround the medical needle, thus preventing reuse. However such latches can be reset and the needle could be reused, which could lead to the spread of disease and infection.

There remains a need for an improved safety needle assembly.

### Summary of the Invention

In an exemplary embodiment, a safety needle assembly according to the present invention comprises a drive unit holding a needle having a distal pointed end, an outer body rotatably coupled to the drive unit, a first tube telescopically coupled to the outer body and the drive unit, and a second tube telescopically coupled to the first tube. In an extended position, the second tube covers the distal pointed end of the needle.
Rotation of the outer body relative to the drive unit causes the first tube and the second tube to retract and expose the distal pointed end.

In an exemplary embodiment, the drive unit includes a disc abutting shoulders in the outer body to prevent axial translation of the drive unit relative to the outer body.

In an exemplary embodiment, the drive unit includes a sleeve having a first notch adapted to engage a first follower pin on the first tube and a second notch adapted to engage a second follower pin on the second tube. The first notch is an at least partially helical channel formed on a first surface of the sleeve and the second notch is an at least partially helical channel formed on a second surface of the sleeve. The first notch includes a distal section adapted to releasably lock the first follower pin until a predetermined rotational force is applied to the first tube by the outer body.

In an exemplary embodiment, the outer body includes a thread adapted to engage a thread on a carpule holder.

In an exemplary embodiment, the drive unit includes a toothing adapted to engage a toothing on a carpule holder.

In an exemplary embodiment, the outer body includes at least one protrusion adapted to engage a longitudinal notch on the first tube.

In an exemplary embodiment, the first tube includes at least one protrusion adapted to engage a longitudinal notch on the second tube.

In an exemplary embodiment, a proximal part of the second tube includes at least one bayonet pin adapted to releasably engage a recess in a distal part of the first tube.

Exemplary embodiments of the safety needle assembly according to the invention can be composed of just a few injection-molded parts and may not need a spring or other metal components but the injection needle. The safety needle assembly according to the invention may operate automatically by screwing it onto the injection device, e.g. a carpule holder for holding a carpule.

Further scope of applicability of the present invention will become apparent from the detailed description given hereinafter. However, it should be understood that the detailed description and specific examples, while indicating preferred embodiments of the invention, are given by way of illustration only, since various changes and modifications within the spirit and scope of the invention will become apparent to those skilled in the art from this detailed description.

### Brief Description of the Drawings

The present invention will become more fully understood from the detailed description given hereinbelow and the accompanying drawings which are given by way of illustration only, and thus, are not limitive of the present invention, and wherein:
- Figure 1: is a longitudinal section of an exemplary embodiment of a safety needle assembly in an initial first position,
- Figure 2A: is a longitudinal section of an exemplary embodiment of a drive unit in a first section plane,
- Figure 2B: is a longitudinal section of an exemplary embodiment of a drive unit in a second section plane,
- Figure 2C: is a perspective view of an exemplary embodiment of a drive unit,
- Figure 3A: is a longitudinal section of an exemplary embodiment of an outer body,
- Figure 3B: is a perspective view of an exemplary embodiment of an outer body,
- Figure 4: is a longitudinal section of an exemplary embodiment of a carpule holder,
- Figure 5A: is a longitudinal section of an exemplary embodiment of an first tube,
- Figure 5B: is a perspective view of an exemplary embodiment of an first tube,
- Figure 6: is a perspective view of an exemplary embodiment of an second tube,
- Figure 7: is a longitudinal section of an exemplary embodiment of a safety needle assembly in a second position,
- Figure 8: is a longitudinal section of an exemplary embodiment of a safety needle assembly in a third position,
- Figure 9: is a longitudinal section of an exemplary embodiment of a safety needle assembly in a fourth position,
- Figure 10: is a longitudinal section of an exemplary embodiment of a safety needle assembly in a fifth position, and
- Figure 11: is a flat projection of exemplary embodiments of drive notches on the drive unit.

Corresponding parts are marked with the same reference symbols in all figures.

### Detailed Description

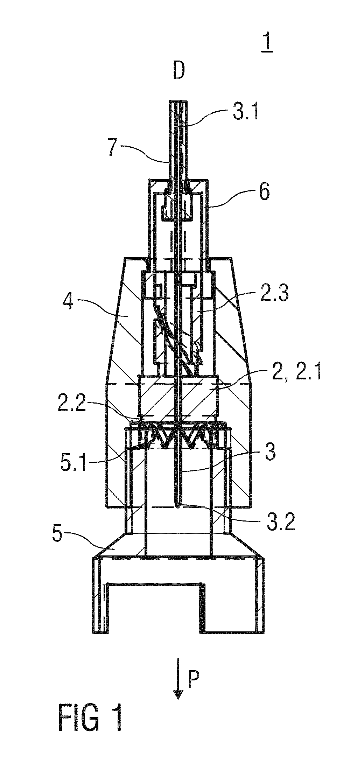

Figure 1 shows an exemplary embodiment of a safety needle assembly 1 according to the present invention in an initial first position prior to use. The safety needle assembly 1 comprises a drive unit 2 holding a hollow injection needle 3 having a pointed distal end 3.1 and a pointed proximal end 3.2. The drive unit 2 is arranged within an outer body 4 in such a manner that the drive unit 2 is axially fixed but the outer body 4 is rotatable relative to the drive unit 2. A first tube 6 is telescoped within the outer body 4 and over a drive unit sleeve 2.3. A second tube 7 is telescoped within the first tube 6.

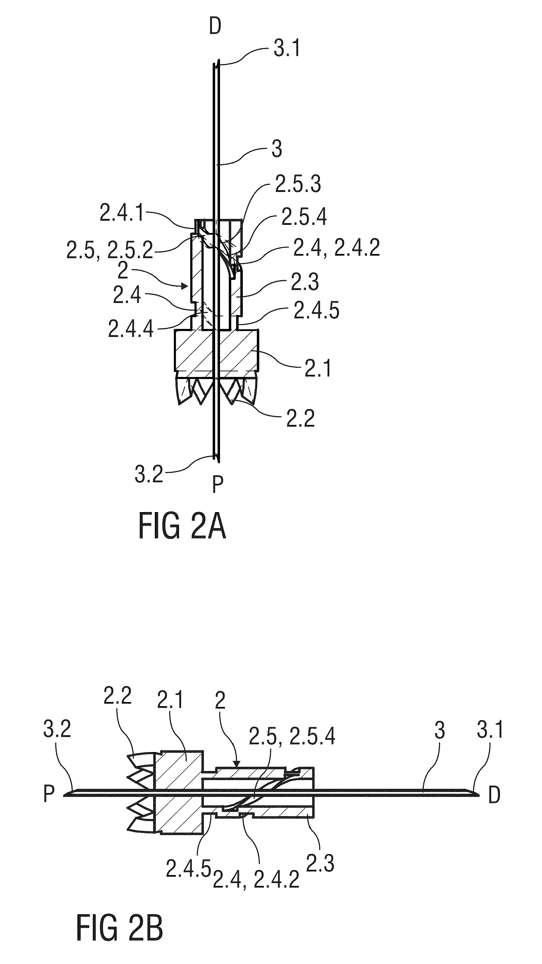

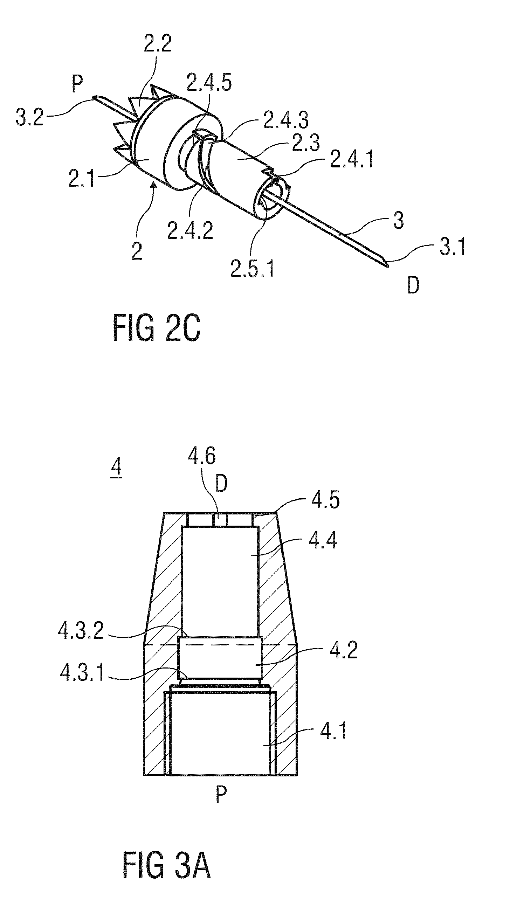

Exemplary embodiments of the drive unit 2 and the needle 3 are shown in detail in figures 2A to 2C. The drive unit 2 comprises a disc 2.1 in which the needle 3 is held. A proximal face of the disc 2.1 exhibits a toothing 2.2. The drive unit 2 further comprises a drive unit sleeve 2.3 extending in the distal direction D from the disc 2.1. The drive unit sleeve 2.3 exhibits a first drive notch 2.4 on an external surface. The first drive notch 2.4 has, in a distal to proximal direction, a distal horizontal notch end section 2.4.1 followed by a first helical section 2.4.2, an intermediate horizontal section 2.4.3, a second helical section 2.4.4 and a proximal horizontal notch end section 2.4.5. The proximal horizontal notch end section 2.4.5 may extend almost 360 degrees of the circumference of the drive unit sleeve 2.3.

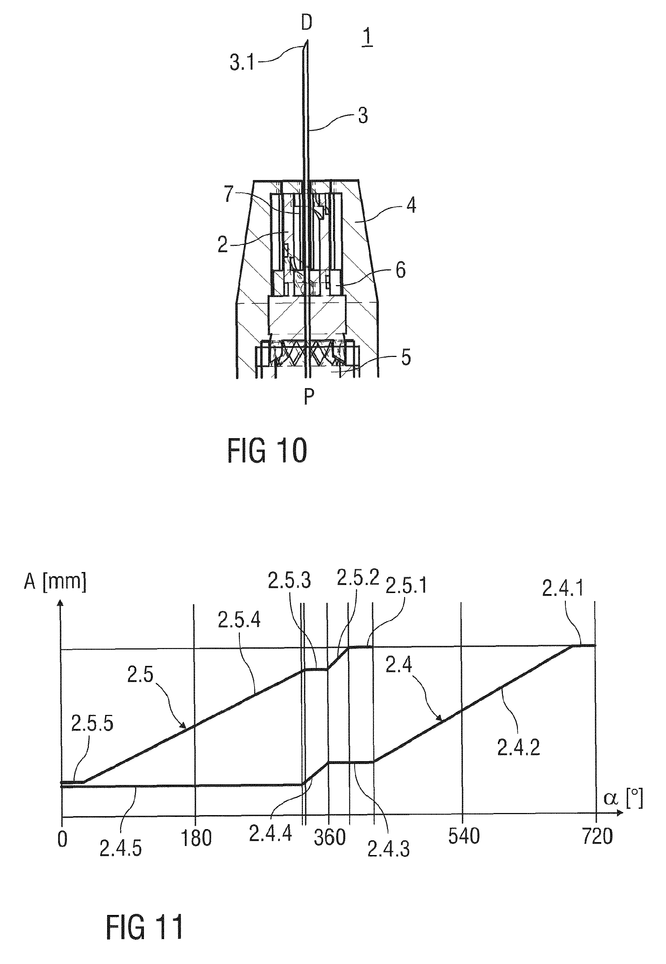

A second drive notch 2.5 formed on an internal surface of the drive unit sleeve 2.3 comprises, in a distal to proximal direction, a distal horizontal lead in 2.5.1 followed by a first helical section 2.5.2, an intermediate horizontal section 2.5.3, a second helical section 2.5.4 and a proximal horizontal notch end section 2.5.5. A flat projection of the first drive notch 2.4 and the second drive notch 2.5 are illustrated in figure 11. The flat projection illustrates an axial position A of the second tube 7 and the first tube 6 depending on their rotation angle α relative to the drive unit 2. The values indicated on the abscissa and the ordinate are given by way of example, only. Those of skill in the art will understand that the drive notches 2.4, 2.5 of the drive unit 2 may be configured in a different way.

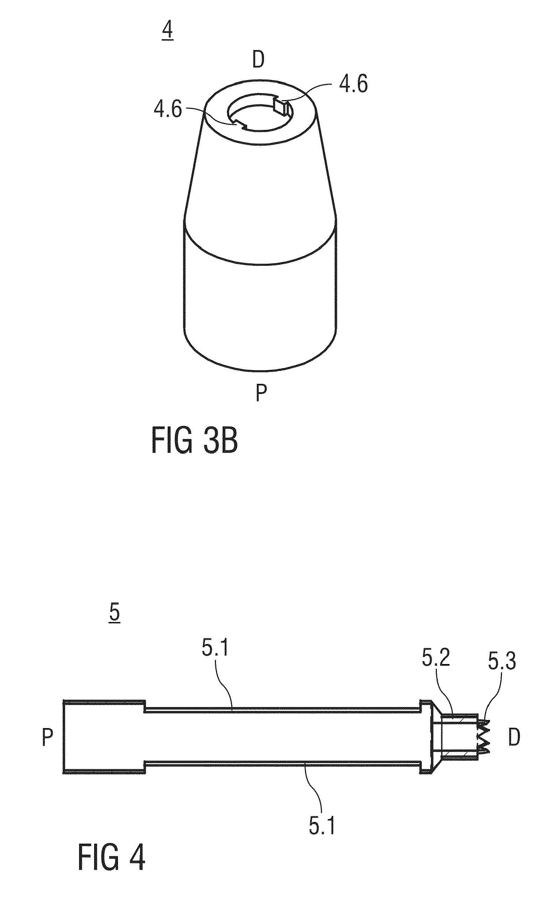

An exemplary embodiment of the outer body 4 is shown in detail in figures 3A and 3B. The outer body 4 has a longitudinal bore with a proximal part 4.1 having a first diameter with an internal thread for screwing the safety needle assembly 1 onto a carpule holder, a center part 4.2 having a second diameter which is adapted to axially retain the disc 2.1 of the drive unit 2 with proximal and distal shoulders 4.3.1, 4.3.2, and a distal part 4.4 having a third diameter smaller than the second diameter. The distal part 4.4 includes a distal end face 4.5 which includes an aperture sized to allow passage of the needle 3. The proximal shoulder 4.3.1 may be resilient and proximally ramped so as to allow insertion of the drive unit 2 into the outer body 4 during assembly and to lock the drive unit 2 in position and prevent it from falling out of the first tube 4. Two radially inwardly directed protrusions 4.6 may be arranged in the distal end face 4.5.

An exemplary embodiment of the carpule holder 5 is shown in detail in figure 4. The carpule holder 5 is essentially sleeve-shaped and arranged to retain a carpule (not illustrated) containing a medicament. Two lateral viewing windows 5.1 in the carpule holder 5 may allow inspection of the medicament. A distal neck part 5.2 of the carpule holder 5 exhibits a reduced diameter for supporting the carpule at its distal end. A distal end of the neck part 5.2 exhibits a toothing 5.3 adapted to engage the toothing 2.2 on the drive unit 2. The carpule comprises a septum at a distal end arranged to be pierced by the pointed proximal end 3.2 of the needle 3 when the safety needle assembly 1 is coupled to the carpule holder 5. In another exemplary embodiment, the toothing 5.3 may be formed on an adapter which can be coupled to a standard medicament delivery device, e.g., a pen injector, an autoinjector, a syringe, etc. For example, such an adapter may clip or releasably lock onto the delivery device and allow the delivery device to utilize the safety needle assembly 1 according to the present invention.

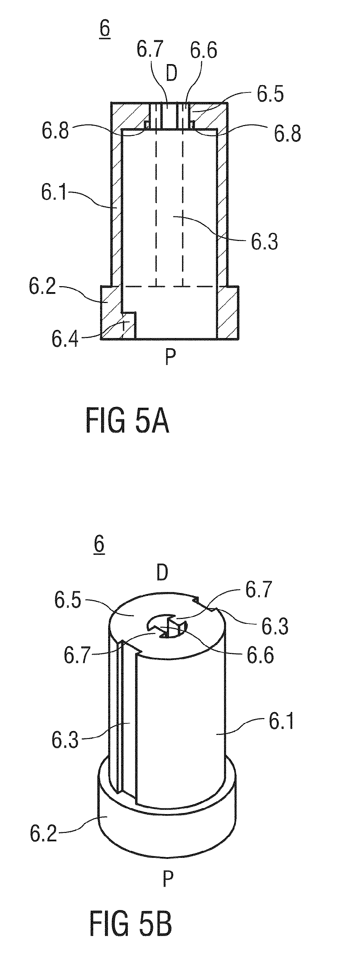

Figures 5A and 5B show an exemplary embodiment of the first tube 6. The first tube 6 has a cylindrical part 6.1 and a proximal collar 6.2. The cylindrical part 6.1 has an external diameter approximately equal to the fourth diameter of the bore in the distal end face 4.5 of the outer body 4. The cylindrical part 6.1 exhibits two longitudinal notches 6.3 adapted to engage the protrusions 4.6 in the outer body 4 so that the first tube 6 can telescope relative to the outer body 4 but is keyed to it for preventing relative rotation. The proximal collar 6.2 has a greater diameter than the cylindrical part 6.1 and is arranged to abut the distal end face 4.5 of the outer body so as to limit their relative axial movement. An internal diameter of the first tube 6 corresponds to an external diameter of the drive unit sleeve 2.3 so that the drive unit 2 can telescope relative to the first tube 6. A radially protruding first follower pin 6.4 on the first tube 6 is arranged to engage the first drive notch 2.4 on the drive unit 2 so that on rotation of the outer body 4 relative to the first tube 6, the first tube 6 translates axially relative to the outer body 4.

A distal end face 6.5 of the first tube 6 comprises a bore 6.6 with a fifth diameter which is smaller than the internal diameter of the cylindrical part 6.1 of the first tube 6. Two radially inwardly directed protrusions 6.7 are arranged within the bore 6.6.

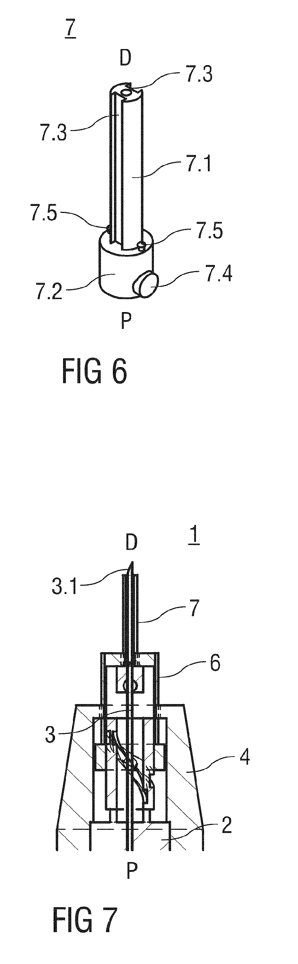

Figure 6 shows an exemplary embodiment the second tube 7. The second tube 7 has a distal part 7.1 and a proximal part 7.2. The distal part 7.1 has an external diameter approximately equal to the fifth diameter of the bore 6.6 in the distal end face 6.5 of the first tube 6. The distal part 7.1 exhibits two longitudinal notches 7.3 for engaging the protrusions 6.7 in the first tube 6 so that the second tube 7 can telescope relative to the first tube 6 but is keyed to it for preventing relative rotation. The proximal part 7.2 has a greater diameter than the distal part 7.1 and is arranged to abut the distal end face 6.5 of the first tube 6 so as to limit their relative axial movement. The diameter of the proximal part 7.2 approximately equals the internal diameter of the drive unit sleeve 2.3 so that the second tube 7 can telescope relative to the drive unit 2. A radially protruding second follower pin 7.4 on the proximal part 7.2 is adapted to engage the second drive notch 2.5 in the drive unit 2 so that on rotation of the second tube 7 relative to the drive unit 2 the second tube 7 translates axially within the second tube 7 once the second follower pin 7.4 has engaged the second drive notch 2.5.

An internal diameter of the second tube 7 is sufficiently wide to allow the needle 3 to move axially within.

In an exemplary embodiment, two mushroom-shaped bayonet pins 7.5 protrude distally from a distal shoulder of the proximal part 7.2. The bayonet pins 7.5 are arranged to engage in corresponding proximal recesses 6.8 in the distal end face 6.5 of the first tube 6.

Referring again to figure 1, the safety needle assembly 1 is shown in a first position prior to use. The second tube 7 is in an extended position relative to the first tube 6 and maintained in the extended position because the bayonet pins 7.5 engage the recesses 6.8 of the first tube 6. The first tube 6 is in an extended position relative to the outer body 4 and the drive unit 2. The first follower pin 6.4 of the first tube 6 is engaged in the distal horizontal notch end section 2.4.1 of the first drive notch 2.4 in the drive unit 2. The first tube 6 is thus releasably locked to the drive unit 2. The second tube 7 is axially spaced from the drive unit 2 so that the second follower pin 7.4 does not engage the second drive notch 2.5. As both the second tube 7 and the first tube 6 are in their respective extended positions, the distal end 3.1 of the needle 3 is covered within the second tube 7.

As the outer body 4 is coupled to the carpule holder 5 (e.g., by screwing), the safety needle assembly 1 may rotate as a whole since the outer body 4 is keyed to the first tube 6 (e.g., by protrusions 4.6 engaged in notches 6.3) and the first tube 6 is keyed to the second tube 7 (e.g., by protrusions 6.7 engaged in notches 7.3). When the toothings 5.3 and 2.2 engage, e.g. two before an end stop of the screw thread between the outer body 4 and the carpule holder 5, the drive unit 2 becomes rotationally locked to the carpule holder 5. As the outer body 4 is further rotated, it will rotate relative to the carpule holder 5 and the drive unit 2. A tactile feedback may be provided in the form of an increased resistance, because the first follower pin 6.4 is engaged in the distal horizontal notch end section 2.4.1. Further rotation of the outer body 4, e.g. one eighth of a turn, causes the first follower pin 6.4 to disengage the distal horizontal notch end section 2.4.1 and enter the first helical section 2.4.2 of the first drive notch 2.4. Further rotation of the outer body 4 thus results in the first follower pin 6.4 travelling down the first drive notch 2.4 in the drive unit 2 so that the first tube 6 is retracted relative to the outer body 4 and the drive unit 2.

Figure 7 shows the safety needle assembly 1 in a second position, during use, with the first follower pin 6.4 having travelled halfway down the first drive notch 2.4. The first tube 6 has partially retracted relative to the outer body 4 and the drive unit 2, and because the second tube 7 is coupled to the first tube 6, the second tube 7 has translated in the proximal direction. In the second position, the distal end 3.1 of the needle 3 may be partially exposed.

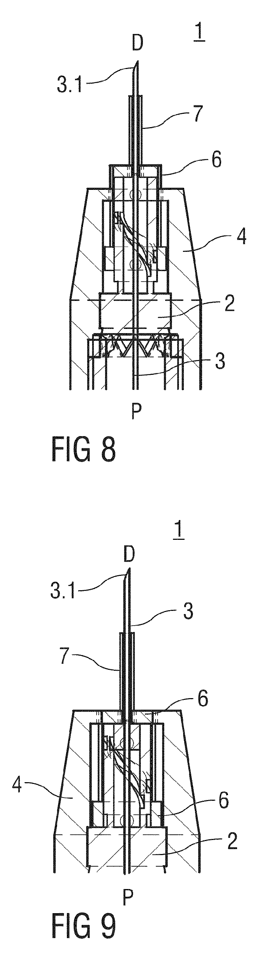

Figure 8 shows the safety needle assembly 1 in a third position during use. As the outer body 4 is rotated relative to the carpule holder 5, the first follower pin 6.4 in the first tube 6 enters the intermediate horizontal section 2.4.3 so that the retraction of the first tube 6 is interrupted, and the second tube 7 is telescoped into the drive unit 2 so that the second follower pin 7.4 on the second tube 7 engages the horizontal lead in 2.5.1 of the second drive notch 2.5.

Figure 9 shows the safety needle assembly 1 in a fourth position during use. On further rotation of the outer body 4, the second follower pin 7.4 on the second tube 7 enters the first helical section 2.5.2 of the second drive notch 2.5 so that the second tube 7 starts retracting within the first tube 6 and the bayonet pins 7.5 disengage the recesses 6.8. Then, the first follower pin 6.4 on the first tube 6 enters the second helical section 2.4.4 in the first drive notch 2.4 and the second follower pin 7.4 on the second tube 7 enters the intermediate horizontal section 2.5.3 so that the retraction of the first tube 6 within the outer body 4 is resumed while the retraction of the second tube 7 within the first tube 6 is interrupted. Eventually, on further rotation of the outer body 4, the second follower pin 7.4 enters the second helical section 2.5.4 and the first follower pin 6.4 on the first tube 6 enters the proximal horizontal notch end section 2.4.5 in the fourth position as illustrated in figure 9. The distal end face 6.5 of the first tube 6 is flush with the distal end face 4.5 of the outer body 4 and remains in this axial position for the rest of the rotation of the outer body 4 while the second tube 7 is retracted further within the first tube 6.

Figure 10 shows the safety needle assembly 1 in a fifth position during use. The distal end of the second tube 7 is flush with the outer body 4 so the needle 3 is fully exposed. The second follower pin 7.4 enters a proximal horizontal notch end section (not illustrated) in the second drive notch 2.5 so that further rotation of the outer body 4 does not move the second tube 7 nor first tube 6 any more. The second tube 7 and the first tube 6 may be fixed in their axial positions and the injection device comprising the carpule holder 5, the carpule and the safety needle assembly 1 may be used to administer an injection.

After an injection, the safety needle assembly 1 may be removed from the carpule holder 5 by unscrewing it. The rotational direction is hence reversed and the second tube 7 and first tube 6 will return to their initial position as in figure 1 guided by the first and second follower pins 6.4 and 7.4 following the first drive notch 2.4 and the second drive notch 2.5, respectively so that all movements described above are performed in reverse.

The bayonet pins 7.5 and the first drive notch 2.4 may comprise features for locking the bayonet pins 7.5 in the recesses 6.8 and the first pin 6.4 in the first drive notch 2.4 after having moved the first tube 6 and second tube 7 into their initial positions when unscrewing the safety needle assembly 1 from the carpule holder 5. Thus, reuse of the safety needle assembly 1 and hence the risk of cross contamination can be prevented.

Those of skill in the art will understand that modifications (additions and/or removals) of various components of the apparatuses, methods and/or systems and embodiments described herein may be made without departing from the full scope and spirit of the present invention, which encompass such modifications and any and all equivalents thereof.

## Claims

1. A safety needle assembly (1) comprising:
a drive unit (2) holding a needle (3) having a distal pointed end (3.1);
an outer body (4) rotatably coupled to the drive unit (2);
a first tube (6) telescopically coupled to the outer body (4) and the drive unit (2); and
a second tube (7) telescopically coupled to the first tube (6),
wherein, in an extended position, the second tube (7) covers the distal pointed end (3.1) of the needle (3), and
wherein, rotation of the outer body (4) relative to the drive unit (2) causes the first tube (6) and the second tube (7) to retract and expose the distal pointed end (3.1).

2. The safety needle assembly (1) according to claim 1, wherein the drive unit (2) includes a disc (2.1) abutting shoulders (4.3.1, 4.3.2) in the outer body (4) to prevent axial translation of the drive unit (2) relative to the outer body (4).

3. The safety needle assembly (1) according to any one of the preceding claims, wherein the drive unit (2) includes a sleeve (2.3) having a first notch (2.4) adapted to engage a first follower pin (6.4) on the first tube (6) and a second notch (2.5) adapted to engage a second follower pin (7.4) on the second tube (7).

4. The safety needle assembly (1) according to claim 3, wherein the first notch (2.4) is an at least partially helical channel formed on a first surface of the sleeve (2.3) and the second notch (2.5) is an at least partially helical channel formed on a second surface of the sleeve (2.3).

5. The safety needle assembly (1) according to claim 3, wherein the first notch (2.4) includes a distal section (2.4.1) adapted to releasably lock the first follower pin (6.4) until a predetermined rotational force is applied to the first tube (6) by the outer body (4).

6. The safety needle assembly (1) according to any one of the preceding claims, wherein the outer body (4) includes a thread adapted to engage a thread on a carpule holder (5).

7. The safety needle assembly (1) according to any one of the preceding claims, wherein the drive unit (2) includes a toothing (2.2) adapted to engage a toothing (5.3) on a carpule holder (5).

8. The safety needle assembly (1) according to any one of the preceding claims, wherein the outer body (4) includes at least one protrusion (4.6) adapted to engage a longitudinal notch (6.3) on the first tube (6).

9. The safety needle assembly (1) according to any one of the preceding claims, wherein the first tube (6) includes at least one protrusion (6.7) adapted to engage a longitudinal notch (7.3) on the second tube (7).

10. The safety needle assembly (1) according to any one of the preceding claims, wherein a proximal part of the second tube (7) includes at least one bayonet pin (7.5) adapted to releasably engage a recess (6.8) in a distal part of the first tube (6).
